# EUROPEAN PATENT SPECIFICATION

(11) **EP 4 653 921 B1**
(45) Date of publication and mention of the grant of the patent: **16.09.2026**
(21) Application number: 25173344.0
(22) Date of filing: 29.04.2025
(51) Int. Cl.: G01T 1/20, G01T 1/29, A61B 6/00, A61B 6/03

(54) **POSITRON EMISSION TOMOGRAPHY (PET) DETECTION COMPONENTS AND SCANNING SYSTEMS**
POSITRONENEMISSIONSTOMOGRAPHIE (PET)-DETEKTIONSKOMPONENTEN UND ABTASTSYSTEME
COMPOSANTS DE DÉTECTION DE TOMOGRAPHIE PAR ÉMISSION DE POSITRONS (PET) ET SYSTÈMES DE BALAYAGE

(30) Priority: 29.04.2024 CN 202420930559 U
(43) Date of publication of application: 26.11.2025
(73) Proprietor: Shanghai United Imaging Healthcare Co., Ltd., Shanghai 201807 (CN)
(72) Inventor: CHEN, Hongyu, Shanghai, 201807 (CN); BI, Dongdong, Shanghai, 201807 (CN); HAN, Zhenjie, Shanghai, 201807 (CN); AN, Shaohui, Shanghai, 201807 (CN)
(74) Representative: Wang, Bo

(56) References cited:
- US-A1- 2007 080 296
- US-A1- 2022 229 195
- US-A1- 2023 045 875

## Description

### CROSS-REFERENCE TO RELATED APPLICATIONS

This application claims priority to Chinese application No. 202420930559.3, filed on April 29, 2024.

### TECHNICAL FIELD

The present application relates to the technical field of medical imaging devices, and in particular, to positron emission tomography (PET) detection components and scanning systems.

### BACKGROUND

Positron Emission Tomography (PET) systems are playing an increasingly important role in the field of medical imaging. The detector, as the core component of a PET system, includes elements such as crystals, photodetectors, and functional chips. The performance of these elements is closely related to the temperature of the detector. A low-temperature environment can enhance the light yield of the crystal, reduce the dark current of the photodetector, improve the operating efficiency of the photodetector, and decrease the operational noise of the functional chips.

Therefore, as exemplified by the cooling system disclosed in US 2022/229195, cooling of the detector is crucial. However, existing heat dissipation systems for detectors often have low cooling efficiency and can result in temperature gradients, leading to poor cooling performance for certain elements within the detector.

### SUMMARY

The present invention provides therefore a positron emission tomography (PET) detection component, comprising at least one PET detector and a heat dissipation device. The PET detector includes an electronic component and crystals that are interconnected. The heat dissipation device includes a shell, a cavity being disposed inside the shell, and a first portion of the PET detector being disposed inside the cavity. The heat dissipation device includes a cooling component, and the cooling component includes a heat-conducting plate and a plurality of cooling members, the heat-conducting plate being located at a bottom of the cavity, the plurality of cooling members being arranged at intervals on the heat-conducting plate, and the plurality of cooling members extending along a height direction of the shell. An inlet and an outlet are disposed on two opposite sides along a short side direction of the shell, respectively, and the inlet and the outlet are in communication with the cavity, the short side direction being a direction of a short side of the shell on a cross-section perpendicular to the height direction. A second portion of the PET detector is connected to a side of the heat-conducting plate back away from the cavity, and the crystals are disposed in the second portion of the PET detector.

One or more embodiments of the present disclosure provide a positron emission tomography (PET) scanning system, comprising a plurality of PET detection components of claim 1. The PET scanning system further comprises a gantry, mounted with the plurality of PET detection components in an array along a circumferential direction of the gantry; a heat dissipation system, configured to deliver a heat dissipation medium to the plurality of PET detection components; and a processor, configured to at least control an operation state of the heat dissipation system.

One or more embodiments of the present disclosure provide a positron emission tomography (PET) detection system. The PET detection system comprises a gantry with a cylindrical structure; a front cover plate, disposed at a front end of the gantry along an axial direction of the cylindrical structure, the front cover plate being provided with an air intake vent; a rear cover plate, disposed at a rear end of the gantry along the axial direction of the cylindrical structure, the rear cover plate being provided with an air outlet vent; a plurality of PET detectors, mounted on the gantry along a circumferential direction of the gantry, each of the plurality of PET detectors extending along a direction from the front cover plate to the rear cover plate; and a heat dissipation device, connected to the plurality of PET detectors. The heat dissipation device includes a shell, the shell forming a cavity, and the cavity being in communication with the air intake vent and the air outlet vent; a cooling component, including a heat-conducting plate and a plurality of cooling members, the heat-conducting plate being thermally coupled with the PET detectors, the plurality of cooling members being arranged at intervals on the heat-conducting plate, and the plurality of cooling members extending along a height direction of the shell; and the cavity forming a flow guide channel for guiding a heat dissipation medium to enter the cavity through the air intake vent to flow along a short side direction of the cavity and flow out of the cavity through the air outlet vent, thereby removing heat from the cooling component; the short side direction being a direction of a short side of the shell on a cross-section perpendicular to the height direction.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering denotes the same structure, wherein:
FIG. 1 is a three-dimensional schematic diagram illustrating a positron emission tomography (PET) detection component according to some embodiments of the present disclosure;
FIG. 2 is a schematic diagram illustrating an exemplary structure of a positron emission tomography (PET) detection component according to some embodiments of the present disclosure;
FIG. 3 is a schematic diagram illustrating a top cross-sectional view of heat dissipation devices according to some embodiments of the present disclosure;
FIG. 4 is a schematic diagram illustrating a top view of heat dissipation devices according to some embodiments of the present disclosure;
FIG. 5 is a schematic diagram illustrating a partially enlarged view of region A in FIG. 4 according to some embodiments of the present disclosure;
FIG. 6 is a schematic diagram illustrating an exemplary structure of a portion of a heat dissipation device according to some embodiments of the present disclosure;
FIG. 7 is a three-dimensional schematic diagram illustrating an air baffle of a heat dissipation device according to some embodiments of the present disclosure;
FIG. 8 is a schematic diagram illustrating a first deflector structure at an inlet according to some embodiments of the present disclosure;
FIG. 9 is a schematic diagram illustrating a partially enlarged view of region B1 and region B2 in FIG. 8 according to some embodiments of the present disclosure;
FIG. 10 is a schematic diagram illustrating an exemplary structure of a second deflector structure at an outlet according to some embodiments of the present disclosure;
FIG. 11 is a schematic diagram illustrating a partially enlarged view of region B3 and region B4 in FIG. 10 according to some embodiments of the present disclosure;
FIG. 12 is a schematic diagram illustrating an exemplary structure of a third deflector structure at an inlet according to some embodiments of the present disclosure;
FIG. 13 is a schematic diagram illustrating an exemplary structure of a fourth deflector structure at an inlet according to some embodiments of the present disclosure;
FIG. 14 is a schematic diagram illustrating an exemplary structure of a positron emission tomography (PET) scanning system according to some embodiments of the present disclosure;
FIG. 15 is a schematic diagram illustrating an exemplary structure of a portion of a heat dissipation system corresponding to one PET detection component according to some embodiments of the present disclosure;
FIG. 16 is a schematic diagram illustrating a side-sectional view of a heat dissipation system according to some embodiments of the present disclosure; and
FIG. 17 is a schematic diagram illustrating an exemplary connection structure of a signal transmission board according to some embodiments of the present disclosure.

### DETAILED DESCRIPTION

The accompanying drawings, which are to be used in the description of the embodiments, are briefly described below. The accompanying drawings do not represent the entirety of the embodiments.

As used herein, "system", "device", "unit" and/or "module" as used herein is a method for distinguishing between different components, elements, parts, sections, or assemblies at different levels. The words may be replaced by other expressions if other words would accomplish the same purpose.

As shown in the present disclosure and the claims, unless the context clearly suggests an exception, the words "a", "an", "one", and/or "the" do not refer specifically to the singular, but may also include the plural. Generally, the terms "including" and "comprising" suggest only the inclusion of clearly identified steps and elements that do not constitute an exclusive list, and the method or device may also include other steps or elements.

When describing the operations performed in the embodiments of the present disclosure, in terms of the steps, the order of the steps is all interchangeable, the steps can be omitted, and other steps can be included in the process of the operation, if not otherwise specified.

In some embodiments, a positron emission tomography (PET) detection component includes at least one PET detector and a heat dissipation device. The PET detector includes an electronic component and crystals that are interconnected. The heat dissipation device includes a shell and a cooling component. A cavity is disposed inside the shell, and a first portion of the PET detector is mounted inside the cavity. The cooling component includes a heat-conducting plate and a plurality of cooling members, the heat-conducting plate being located at a bottom of the cavity, the plurality of cooling members being arranged at intervals on the heat-conducting plate, and the plurality of cooling members extending along a height direction of the shell. An inlet and an outlet are disposed on two opposites along a short side direction of the shell, respectively, the inlet and the outlet are in communication with the cavity, and the short side direction is a direction of a short side of the shell on a cross-section perpendicular to the height direction. A second portion of the PET detector is connected to a side of the heat-conducting plate back away from the cavity, and the crystals are disposed in the second portion of the PET detector.

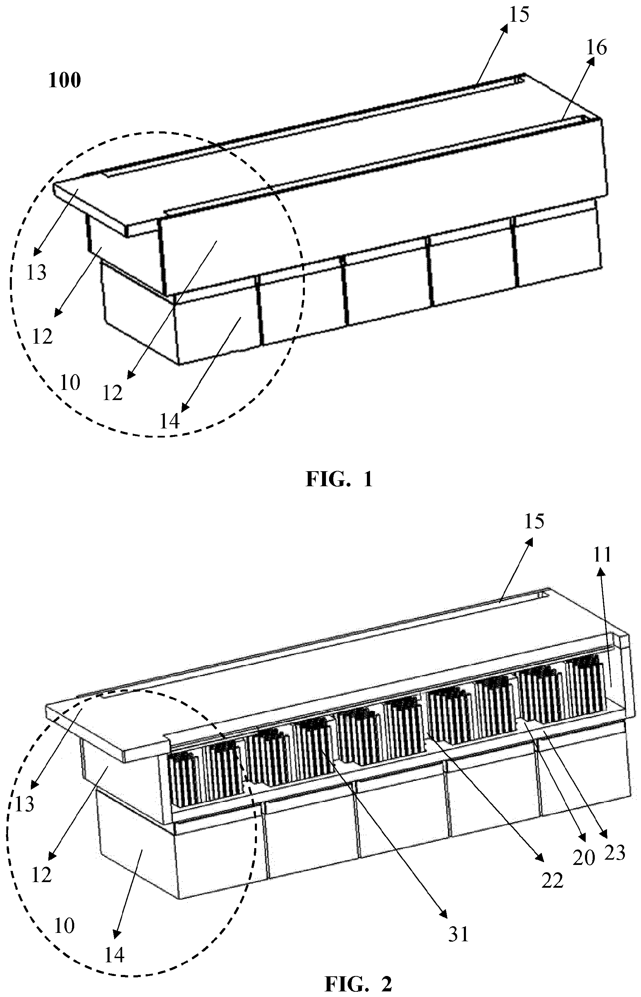

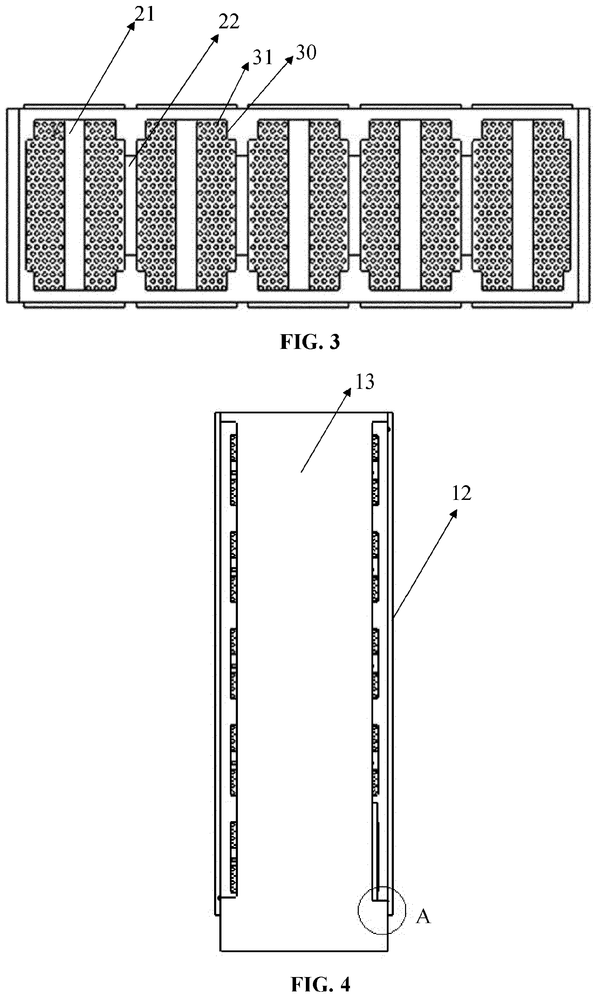

FIG. 1 is a three-dimensional schematic diagram illustrating an exemplary structure of a PET detection component according to some embodiments of the present disclosure. FIG. 2 is a schematic diagram illustrating an exemplary structure of a PET detection component according to some embodiments of the present disclosure. FIG. 3 is a schematic diagram illustrating a top cross-sectional view of heat dissipation devices according to some embodiments of the present disclosure.

The PET detection component refers to a detector component suitable for a Positron Emission Tomography (PET) system, consisting of one or more PET detectors and a heat dissipation device.

The heat dissipation device includes a shell and a cooling component.

As shown in FIG. 1 and FIG. 2, the shell 10 includes a side plate 12, a top plate 13, and a cover shell 14, and a cavity 11 is disposed inside the shell 10. The cooling component includes a heat-conducting plate 20 and a plurality of cooling members 31. The heat-conducting plate 20 is disposed at the bottom of the cavity 11, the plurality of cooling members 31 are disposed at intervals on the heat-conducting plate 20, and the plurality of cooling members 31 extend along a height direction along the shell. The top surface of the cavity 11 is the top plate 13, the bottom surface of the cavity 11 is the heat-conducting plate 20, and the side surface of the cavity 11 is the side plate 12.

The PET detector is configured to receive gamma rays to generate a detection signal, and the PET detector includes an electronic component and crystals that are interconnected.

The electronic component includes a circuit board 23 illustrated in the figures, as well as other electronic elements not shown in the figures (e.g., a photoconverter and a function chip). The crystals are used to detect high-energy gamma photons. When gamma photons interact with the crystals (through Compton scattering or the photoelectric effect), the crystals absorb the energy and emit scintillation light (visible light or ultraviolet light). This scintillation light is then converted into an electrical signal by a photodetector. The material type of the crystal may be NaI (sodium iodide), BGO (bismuth germanate), LSO (lutetium silicate), etc.

A detailed description of a connection relationship between the electronic component and the crystals, and an arrangement relationship between the PET detector and the heat dissipation device can be referred to the descriptions below.

In some embodiments, as shown in FIG. 2, the shell 10 includes a plurality of side plates 12, the top plate 13 is disposed over the top of the plurality of side plates 12, the heat-conducting plate 20 is connected to the bottom of the plurality of side plates 12, and a gap exists between free ends of the plurality of cooling members 31 away from the heat-conducting plate 20 and the top plate 13.

As shown in FIG. 1 and FIG. 2, a count of the side plate 12 may be four, including two smaller left and right side plates arranged along a width direction of the shell 10 and two larger front and rear side plates arranged along a length direction of the shell 10, i.e., the left side plate and the right side plate are side plates formed by the width direction and the height direction of the shell 10, and the front side plate and the rear side plate are side plates formed by the length direction and the height direction of the shell 10. The width direction is the same as the short side direction, and the short side direction refers to a direction of a short side of the shell on a cross-section perpendicular to the height direction. The length direction is the same as a long side direction, and the long side direction refers to a direction of a long side of the shell on the cross-section perpendicular to the height direction.

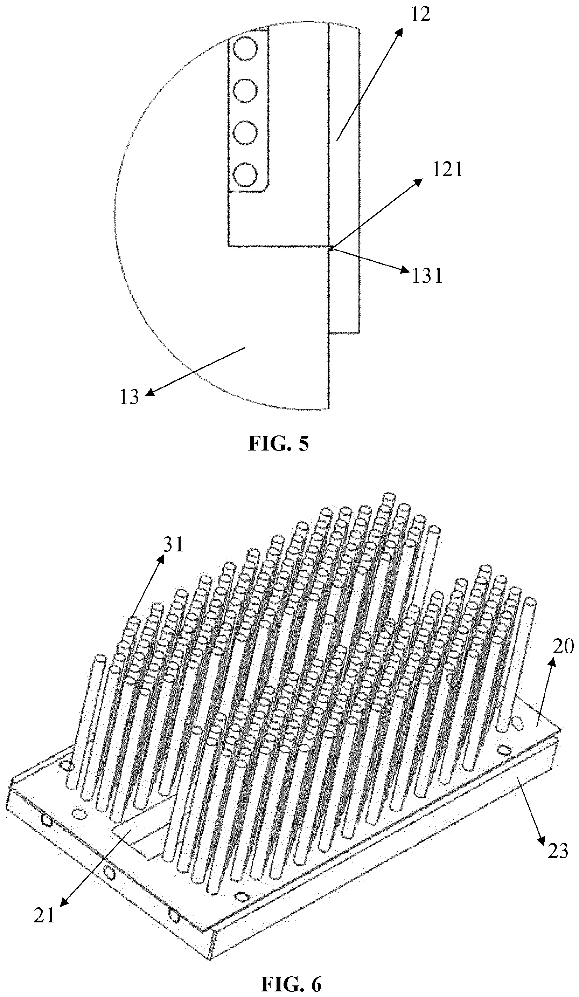

FIG. 4 is a schematic diagram illustrating a top view of heat dissipation devices according to some embodiments of the present disclosure. FIG. 5 is a schematic diagram illustrating a partially enlarged view of region A in FIG. 4 according to some embodiments of the present specification.

As shown in FIG. 4 and FIG. 5, one of the top plate 13 or the side plate 12 is provided with an assembly protrusion 131, and another of the top plate 13 or the side plate 12 is provided with an assembly groove 121, and the assembly protrusion 131 is embedded in the assembly groove 121 to improve the connection strength and sealing between the top plate 13 and the side plate 12 and prevent leakage of a heat dissipation medium inside the cavity 11.

In some embodiments, the assembly protrusions 131 are disposed on two sides along a width direction of the top plate 13, respectively, and the assembly grooves 121 are disposed on two sides along a width direction of the side plate 12 (i.e., the left side plate and the right side plate), respectively, thereby realizing a stable connection between the side plate 12 and the top plate 13. As another example, the assembly grooves 121 are disposed on two sides along the width direction of the top plate 13, respectively, and the assembly protrusions 131 are disposed on two sides along the width direction of the side plate 12 (i.e., the right side plate and the left side plate), respectively. In this way, the side plate 12 is seamlessly connected to the top plate 13 through the snap-fit of the assembly protrusions 131 and the assembly grooves 121.

In some embodiments, the top plate 13 and the side plate 12 may also be connected together by means of adhesion or welding, rather than being limited to the snap-fit of the assembly protrusions 131 and the assembly grooves121 as described above.

In some embodiments of the present disclosure, a reasonable gap is reserved between the cooling members and the top plate along the height direction of the shell, which can prevent short circuits caused by the cooling members being too close to the top plate, or insufficient length (i.e., small surface area) of the cooling members, which would result in reduced airflow heat exchange and poor heat dissipation performance.

The cavity 11 refers to a heat dissipation cavity including the side plate 12, the top plate 13, and the heat-conducting plate 20.

In some embodiments, the cavity includes a plurality of sub-cavities, the plurality of sub-cavities are separated from each other, and the plurality of sub-cavities are disposed along a long side direction of the shell. The heat-conducting plate is equipped with a plurality of groups of heat dissipation units, each group of heat dissipation units includes at least one cooling member, and at least two groups of heat dissipation units are disposed in each sub-cavity, and any one of the plurality of sub-cavities is in communication with an inlet and an outlet.

The cooling member refers to a member used for heat exchange. In some embodiments, the cooling member may be provided as a cylindrical structure, a prismatic structure, a plate-like structure, or other feasible structures. For example, the cooling member may be provided as a four-pronged, six-pronged, or eight-pronged structure, depending on the work requirements.

In some embodiments, each group of heat dissipation units 30 includes a plurality of arrays of cooling members 31 arranged in a staggered manner, as illustrated in FIG. 2 and FIG. 3. A plurality of groups of heat dissipation units 30 are disposed on the heat-conducting plate 20, and the plurality of groups of heat dissipation units 30 are disposed along the long side direction of the shell 10.

In some embodiments, in conjunction with FIG. 2 and FIG. 6, each cooling member 31 may be fixedly connected to a surface of the heat-conducting plate 20 facing the top plate 13 (i.e., a surface in contact with the cavity 11), or other connection manners such as adhesion or welding may be used. A surface of the heat-conducting plate 20 opposite to the top plate 13 (i.e., a surface away from the cavity 11) is connected to the circuit board 23 with connection manners including but not limited to fixed connection using screws. FIG. 6 is a schematic diagram illustrating an exemplary structure of a portion of a heat dissipation device according to some embodiments of the present disclosure.

The heat-conducting plate 20 is made of a high thermal conductivity material, such as aluminum carbide, silicon nitride, or copper, to enable the rapid transfer of heat from the circuit board 23 to the cooling member 31, thereby effectively lowering the temperature of the cooling member 31.

In some embodiments of the present disclosure, by arranging the plurality of arrays of cooling members 31 in a staggered manner to form the heat dissipation unit 30, and reserving a gap between adjacent cooling members 31, the heat dissipation medium can flow through the gap to increase heat exchange efficiency between the cooling members 31. Further, the cooling members 31 arranged in a staggered manner can allow the heat transfer medium to flow turbulently between the cooling members 31, enhancing heat exchange efficiency. In addition, by arranging the plurality of groups of heat dissipation units 30 on the heat-conducting plate 20, with each group of heat dissipation units 30 corresponding to a heat-generating region on the circuit board 23, targeted cooling of specific regions on the circuit board 23 can be realized.

In some embodiments, as shown in FIG. 3 and FIG. 6, the heat-conducting plate 20 is provided with at least one through-slot 21, the at least one through-slot 21 is arranged along the long side direction of the shell 10, the through-slot 21 extends along the short side direction of the shell 10, two sides of the through-slot 21 are equipped with at least one group of heat dissipation units 30, respectively, and a first portion of the PET detector is disposed in the through-slot 21.

The first portion of the PET detector refers to a portion of the PET detector located within the cavity, including an upper horizontal plate and a vertical plate. The upper horizontal plate may be a circuit board (not shown in the figures) connected to one surface of the top plate in contact with the cavity 11. The through-slot 21 is used for the circuit board connected to the surface of the heat-conducting plate 20 back away from the cavity 11 to be connected to the other electronic elements of the PET detector. Specifically, the vertical plate passes through the through-slot 21 on the heat-conducting plate 20 and is connected upwardly to the upper horizontal plate along the height direction of the shell, and is connected downwardly to the circuit board attached to the surface of the heat-conducting plate back away from the cavity, and the vertical plate may also be a circuit board.

In some embodiments, the through-slot 21 may be configured as a rectangular shape and extend along the width direction of the heat-conducting plate 20. The rectangular shape of the through-slot 21 facilitates processing and makes it easier to assemble and connect with other elements.

In some embodiments, a plurality of air baffles 22 are disposed inside the cavity 11, as shown in FIG. 2 and FIG. 3, and at least one of the plurality of air baffles 22 is disposed between any two adjacent sub-cavities.

The placement direction of each air baffle 22 is along the short side direction of the shell, and the plurality of air baffles 22 are arranged along the long side direction of the shell. The cavity 11 is divided by the plurality of air baffles 22 into a plurality of sub-cavities arranged along the long side direction of the shell.

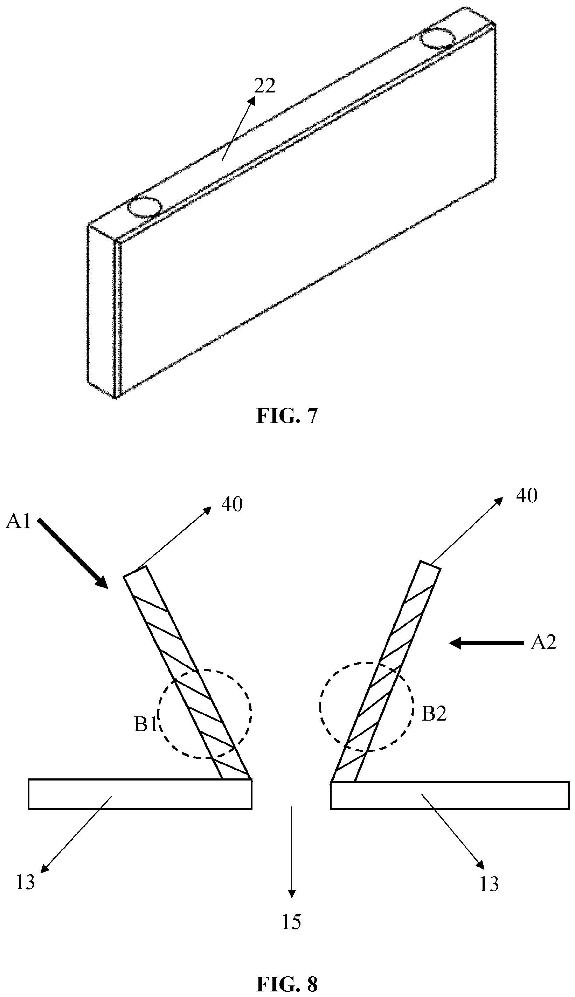

In some embodiments, the air baffle 22 may be configured as a rectangular block, with its shape adapted to fit a gap between adjacent heat dissipation units 30, as shown in FIG. 7. FIG.7 is a three-dimensional schematic diagram illustrating an air baffle of a heat dissipation device according to some embodiments of the present disclosure.

In some embodiments, the air baffle 22 may also be configured as an inclined plate structure or a curved surface structure, etc., to direct a heat dissipation medium toward the heat dissipation unit 30 (i.e., the gap between adjacent cooling members 31).

The heat dissipation medium may be air or coolant, i.e., the heat dissipation device may be configured as an air-cooled heat dissipation device or a liquid-cooled heat dissipation device.

In some embodiments of the present disclosure, the air baffle 22 can fill the gaps between the heat dissipation units 30, effectively preventing the heat dissipation medium from passing through the gaps between adjacent heat dissipation units 30. Further, due to the array arrangement of the plurality of cooling members 31, there are more gaps formed between the plurality of cooling members 31, which forces the heat dissipation medium to pass through the gaps between the plurality of cooling members 31, thereby improving the heat exchange efficiency between the plurality of cooling members 31.

In some embodiments, the cavity 11 may be divided into sub-cavities using various structures, which is not limited to the air baffle 22 described above.

In some embodiments, as shown in FIG. 1 and FIG. 2, the heat dissipation device further includes a cover shell 14, the cover shell 14 covering a side of the heat-conducting plate 20 back away from the cavity 11, and a second portion of the PET detector is disposed in the cover shell 14, and the second portion of the PET detector is connected to the heat-conducting plate 20.

The side of the heat-conducting plate 20 back away from the cavity 11 is connected to the cover shell 14 to form an accommodation cavity, i.e., the accommodation cavity is a cavity formed by the circuit board 23 connected to the side of the heat-conducting plate 20 back away from the cavity 11 and the cover shell 14.

The second portion of the PET detector includes the circuit board 23 connected to the side of the heat-conducting plate 20 back away from the cavity 11 and a plurality of crystals connected to a side of the circuit board 23 back away from the heat-conducting plate 20.

The first portion of the PET detector is disposed inside the cavity 11, and the second portion of the PET detector is connected to the side of the heat-conducting plate 20 back away from the cavity 11, i.e., a plurality of coolers are disposed inside the cavity 11 (a heat dissipation cavity), and a plurality of crystals are disposed inside the accommodation cavity.

In some embodiments of the present disclosure, by disposing the cover shell 14 on the side of the heat-conducting plate 20 back away from the cavity 11, a surface of the heat-conducting plate 20 facing away from the top plate is protected and a mounting space for accommodating electronic elements of the PET detector is formed.

In some embodiments, an inlet and an outlet are disposed on two sides along the short side direction of the shell, respectively, the inlet and the outlet are in communication with the cavity, and the short side direction refers to a direction of a short side of the shell on a cross-section perpendicular to the height direction.

According to the present invention, an inlet 15 and an outlet 16 are located on two opposite long sides of the top plate 13, respectively, and the inlet 15 and the outlet 16 are configured as an elongated shape, as shown in FIG. 1 and FIG. 2. Both the inlet 15 and the outlet 16 are opened on the top plate 13, so that the heat dissipation medium flows in from the top plate 13, and finally flows out from the top plate 13, which can form a U-shaped flow path inside the cavity 11, and improve the heat dissipation efficiency.

In some embodiments, the inlet 15 and the outlet 16 may also be opened on two opposite left and right side plates 12, respectively, and may be configured as a rectangular shape, a circular shape, or any other feasible shapes.

In some embodiments, one of the inlet 15 or the outlet 16 may also be opened on the long side of the top plate 13 and another may be opened on the left side plate 12 or the right side plate 12, and may be configured as a rectangular shape, a circular shape, or any other feasible shape.

The inlet 15 refers to an inlet for the heat dissipation medium to flow into the cavity 11 and the outlet 16 refers to an opening for the heat dissipation medium to flow out of the cavity 11. The cavity 11 is divided into a plurality of sub-cavities, and the inlet 15 and the outlet 16 are in communication with the plurality of sub-cavities.

The shell 10 includes a plurality of side plates 12 and the top plate 13, the top plate 13 is disposed over the top of the plurality of side plates 12, the heat-conducting plate 20 is connected to the bottom of the plurality of side plates 12, and the plurality of cooling members 31 extend along a direction toward the top plate 13, and ends of the cooling members 31 proximate to the top plate 13 are spaced from the top plate 13. In this way, the shell 10 is formed by assembling the plurality of side plates 12 and the top plate 13, and the top plate 13 and the plurality of side plates 12 are processed separately and then assembled together, which reduces process complexity, eliminating the need for direct one-piece fabrication of the shell 10, and also facilitates the installation of other elements within the cavity 11. Ends of the cooling members 31 proximate to the top plate 13 are spaced apart from the top plate 13, which facilitates the installation of the cooling members 31 and avoids interference between the cooling members 31 and the top plate 13.

The shell 10 is provided with the inlet 15 and the outlet 16, so that the heat dissipation medium can flow into the cavity 11 from the inlet 15, exchange heat with the heat-conducting plate 20, and then flow out of the cavity 11 from the outlet 16, and take away the heat from the heat-conducting plate 20, thereby cooling the heat-conducting plate 20. The heat-conducting plate 20 is provided with a plurality of cooling members 31, the plurality of cooling members 31 may distribute the heat load on the heat-conducting plate 20 and increase the contact area between the heat-conducting plate 20 and the heat dissipation medium, thereby improving the heat transfer efficiency per unit time and enhancing the heat exchange performance. The heat of the heat-conducting plate 20 is mainly transferred by the circuit board 23 connected to the side of the heat-conducting plate back away from the cavity 11.

Alternatively, since the heat dissipation medium flows into the cavity 11 from the inlet 15 at a relatively low temperature, the heat exchange efficiency between the heat dissipation medium, the cooling members 31 and the heat-conducting plate 20 is high at this stage, and the temperature of the heat-conducting plate 20 is low. As the heat dissipation medium exchanges heat with the heat-conducting plate 20, the temperature of the heat dissipation medium gradually increases. Therefore, a temperature difference between a side of the heat-conducting plate 20 near the outlet 16 of the shell 10 and the heat dissipation medium becomes smaller, leading to a decrease in the heat exchange efficiency. As a result, a temperature gradient appears on the heat-conducting plate 20. The inlet 15 and the outlet 16 are opened on two sides along the short side direction of the shell 10, respectively, which means that the circulation path of the heat dissipation medium is very short, which not only effectively reduces the resistance caused by the cooling members 31 to the flow of the heat dissipation medium but also minimizes the temperature gradient on the heat-conducting plate 20. In addition, dividing the cavity 11 into a plurality of sub-cavities enables more effective heat exchange of the heat-conducting plate 20 and the cooling members 31 in the plurality of sub-cavities, thereby improving heat dissipation efficiency.

According to the present invention, by opening the inlet 15 and the outlet 16 along the short side direction of the shell 10, the heat of the heat-conducting plate 20 disposed in the shell 10 (i.e., the heat generated by the circuit board 23) is taken away by the flow of the heat dissipation medium between the inlet 15 and the outlet 16, thereby shortening the flow path of the heat dissipation medium, minimizing the temperature gradient on the circuit board 23, and increasing the heat dissipation efficiency as well as the operation capability of the PET detector. The structure and the layout of the cooling members 31 on the heat-conducting plate 20 are also optimized to further improve the heat exchange speed between the cooling members 31 and the heat dissipation medium.

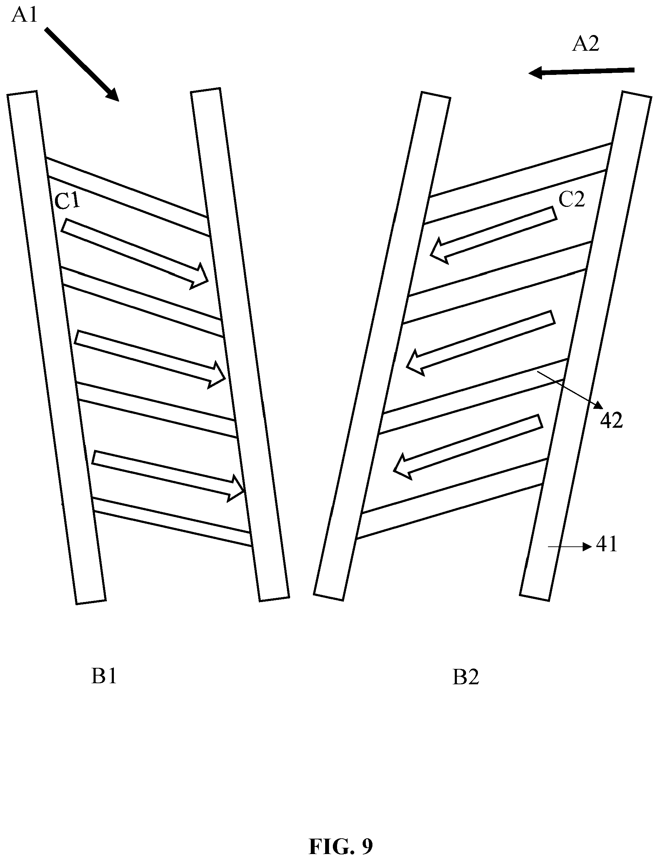

FIG. 8 is a schematic diagram illustrating a first deflector structure at an inlet according to some embodiments of the present disclosure. FIG. 9 is a schematic diagram illustrating a partially enlarged view of region B1 and region B2 in FIG. 8 according to some embodiments of the present disclosure.

In some embodiments, an outer opening of the inlet 15 on an outer side wall of the top plate 13 is provided with a trumpet-shaped first deflector structure, and the outer opening of the inlet is located at a small end of the trumpet-shaped first deflector structure.

The first deflector structure is considered to be trumpet-shaped, i.e., a conical structure that gradually narrows from the large end to the small end. The small end refers to the end with a smaller opening size, while the large end refers to the end with a larger opening size.

In some embodiments, as shown in FIG. 8 and FIG. 9, the first deflector structure includes two deflector plates 40, the two deflector plates 40 are disposed opposite to each other so that the first deflector structure is trumpet-shaped, and each of the deflector plates 40 includes a frame 41 and a plurality of deflector blades 42 arranged in parallel, and the plurality of deflector blades 42 are rotationally connected to the frame 41, and both of the two deflector plates 40 are rotationally connected to the top plate 13.

The first deflector structure is trumpet-shaped with a small end facing downward, consisting of two deflector plates on the left and right. The inlet 15 is disposed at the small end of the first deflector structure and is configured to deflect and collect airflow in multiple directions.

As shown in FIG. 8 and FIG. 9, for the inlet 15, a deflector channel C1 and a deflector channel C2 point to the inlet 15, so the deflector plates 40 can increase the incoming airflow and enhance the cooling effect. The airflow originally along a direction A2 may not enter the inlet 15. However, after being deflected through the deflector channel C2, the direction of the airflow is changed to C2, allowing it to enter the inlet 15 and increasing the intake volume. The airflow originally along a direction A2 may only allow a small portion to enter the inlet 15. However, after being deflected through the flow channel C1, a portion of the airflow that would not have entered the inlet 15 is deflected towards the inlet 15, increasing the intake volume.

In some embodiments, for the first deflector structure at the inlet, the plurality of deflector blades are all rotationally connected to the frame and the two deflector plates are all rotationally connected to the top plate, i.e., an inlet direction can be adjusted by rotating the deflector blades and the deflector plates to selectively collect the airflow in the specified direction to adjust an additional intake volume. The additional intake volume refers to the amount of gas that enters the inlet in the region beyond the angular region (the trumpet-shaped region) between the two deflector plates.

In some embodiments, for the first deflector structure at the inlet, if the deflector blades are set horizontally, they do not collect airflow in the horizontal direction, thus preventing interference with the intake of adjacent heat dissipation devices. Due to the arrangement of arrays of a plurality of heat dissipation devices, the heated air discharged from an outlet of one of the plurality of heat dissipation devices may re-enter an outlet of an adjacent heat dissipation device. By using the deflector blades to close the deflector channels, the airflow outside the trumpet-shaped region is minimized, allowing only the airflow between the two flow deflector plates to be collected, which prevents the heated air from other heat dissipation devices from entering the inlet and affecting the heat dissipation efficiency.

In some embodiments, the rotation angle of the deflector blades and the rotation position of the deflector plates of the first deflector structure may be adjusted according to the actual situation.

For example, when the airflow volume of the PET detection component is low, the wind speed is low, and the temperature of the PET detector is high: the angle between the two deflector plates at the inlet can be increased and the corresponding trumpet-shaped region can be enlarged to increase the intake airflow. Additionally, by rotating the deflector blades to direct the deflector channel towards the inlet, the intake airflow is further increased.

As another example, if the airflow volume of the PET detection component is large, the wind speed is high, and the temperature of the PET detector is low: the angle between the two deflector plates at the inlet can be reduced and the corresponding trumpet-shaped region can be decreased to maintain the intake airflow. Additionally, by rotating the deflector blades to make the deflector blades fit tightly, the deflector channel is closed, thereby maintaining the intake airflow.

In some embodiments of the present disclosure, the direction and size of the deflector channel between the deflector blades are adjusted to control the intake volume at the inlet, with increasing the intake airflow at the inlet and enhancing the cooling effect, while decreasing the intake airflow and maintaining the cooling effect.

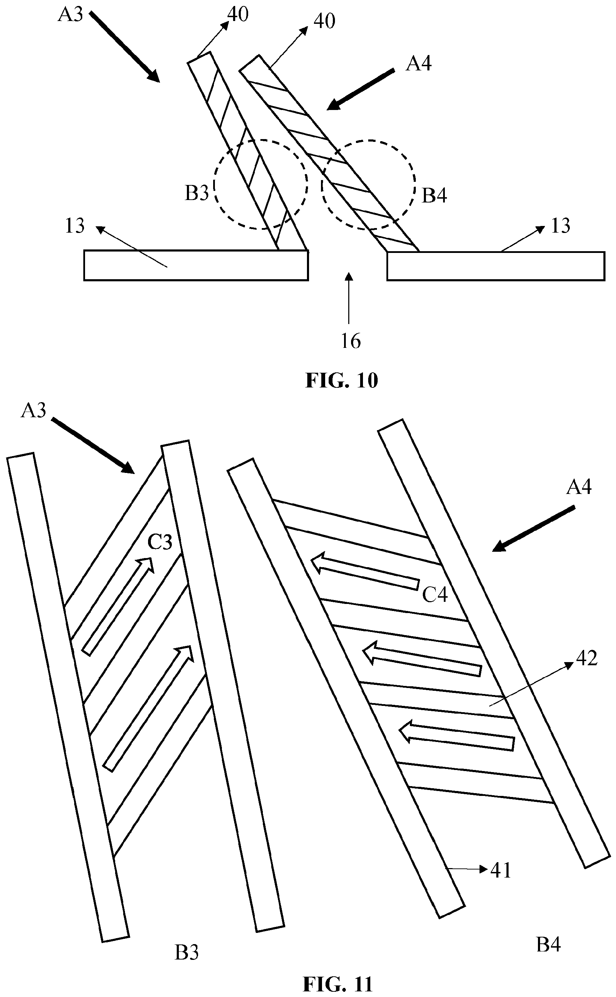

FIG. 10 is a schematic diagram illustrating a second deflector structure at an outlet according to some embodiments of the present disclosure. FIG. 11 is a schematic diagram illustrating a partially enlarged view of region B3 and region B4 in FIG. 10 according to some embodiments of the present disclosure.

In some embodiments, as shown in FIG. 10, an outer opening of the outlet 16 on an outer side wall of the top plate 13 is provided with a trumpet-shaped second deflector structure, and the outer opening of the outlet 16 is located at a large end of the trumpet-shaped second deflector structure.

In some embodiments, as shown in FIG. 10 and FIG. 11, the second deflector structure includes two deflector plates 40, the two deflector plates 40 are disposed opposite to each other so that the second deflector structure is trumpet-shaped, and each of the deflector plates 40 includes the frame 41 and a plurality of deflector blades 42 arranged in parallel, the plurality of deflector blades 42 are also rotationally connected to the frame 41, and both of the deflector plates 40 are rotationally connected to the top plate 13.

The second deflector structure is trumpet-shaped with a large end facing downward, including two deflector plates on the left and right. The outlet 16 is disposed at the large end of the second deflector structure and is configured to prevent air from entering the outlet as much as possible.

As shown in FIG. 10 and FIG. 11, for the outlet 16, a deflector channel C3 and a deflector channel C4 are back away from the outlet 16, so the deflector plates 40 can prevent the air from entering the outlet 16 as much as possible. The airflow originally along a direction A3 may have some portion entering the outlet 16. However, after being deflected through the deflector channel C3, the direction of the airflow is changed to C3, diverting it away from the outlet 16 or preventing it from entering the channel to minimize the intake at the outlet 16. The airflow originally along a direction A4 may have some portion entering the outlet 16. However, after being deflected through the deflector channel C4, the direction of the airflow is changed to C4, diverting it away from outlet 16 to minimize the intake at the outlet 16.

In some embodiments, for the second deflector structure at the outlet, the plurality of deflector blades are all rotationally connected to the frame, i.e., the deflector blades may be rotated to prevent the air from entering the outlet and to avoid affecting the heat dissipation, and the two deflector plates are all rotationally connected to the top plate, allowing the direction of the airflow at the outlet to be adjusted by rotating the deflector plates, thereby preventing the heated air from flowing towards other heat dissipation devices.

In some embodiments, for the second deflector structure at the outlet, if the deflector blades are tightly fitted together, it prevents the air from passing through the deflector plates into the outlet. By adjusting the deflector blades to a suitable position (i.e., the direction of the deflector channel is back away from the outlet), and the air outside the deflector plate can be deflected away from the outlet.

In some embodiments, the rotation angle of the deflector blades and the rotation position of the deflector plates of the second deflector structure may also be adjusted according to the actual situation.

For example, if the airflow volume of the PET detection component is low, the wind speed is low, and the temperature of the PET detector is low: the angle between the two deflector plates at the outlet can be decreased and the corresponding trumpet-shaped region can be reduced to ensure the airflow while minimizing the entry of external air into the outlet.

As another example, if the airflow volume of the PET detection component is large, the wind speed is high, and the temperature of the PET detector is high: the angle between the two deflector plates at the outlet can be increased and the corresponding trumpet-shaped region can be increased to ensure the airflow volume, thereby maintaining the cooling effect.

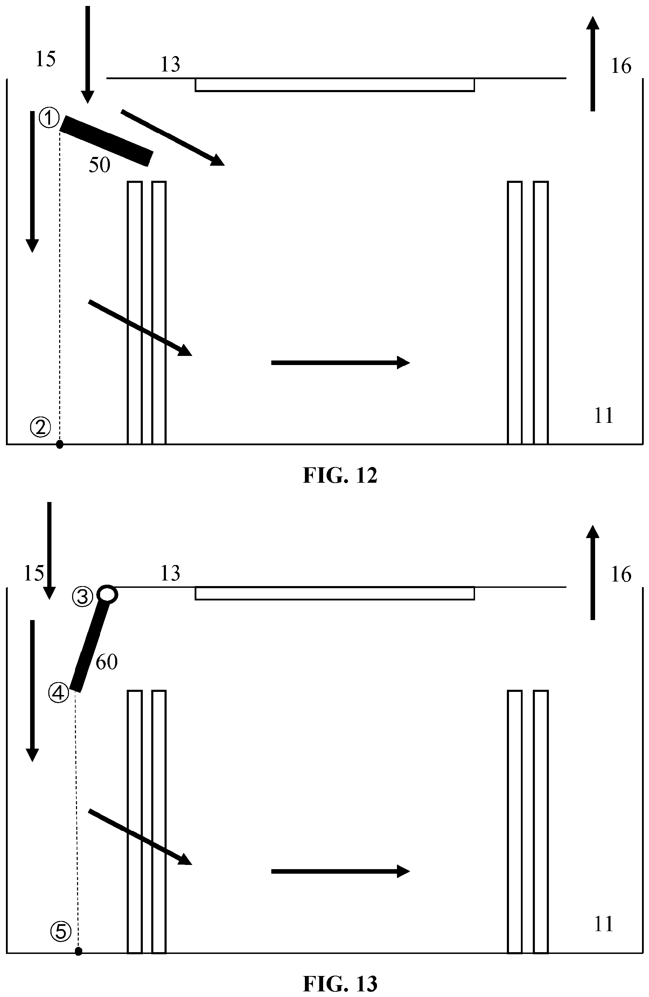

FIG. 12 is a schematic diagram illustrating a third deflector structure at an inlet according to some embodiments of the present disclosure. The black rectangle in FIG. 12 denotes a third deflector structure 50, where ① denotes an end of the third deflector structure 50, ② denotes a projection of the end ① along a height direction, the thick black arrows denote the airflow, and the arrows point in the direction of the airflow.

In some embodiments, as shown in FIG. 12, the third deflector structure 50 is disposed in the cavity 11, the projection ② of the end ① of the third deflector structure 50 is located within a projection of the inlet 15 along the height direction.

In some embodiments, an angle between the third deflector structure 50 and the top plate 13 may be determined according to the actual requirements, ensuring that the third deflector structure does not intercept too much cooling air while ensuring that enough cooling air flows through the cooling members in the cavity.

In some embodiments of the present disclosure, a third deflector structure is provided in the vicinity of the top plate, which facilitates heat dissipation of electronic elements on the top plate.

FIG. 13 is a schematic diagram illustrating a fourth deflector structure at an inlet according to some embodiments of the present disclosure. The black rectangle in FIG. 13 denotes a fourth deflector structure 60, where ③ denotes one end of the fourth deflector structure 60 connected to a resilient pivot shaft (denoted by the circle in the figure), ④ denotes another end of the fourth deflector structure 60, ⑤ denotes a projection of the end ④ along a height direction, and the thick black arrows denote the airflow, and the arrows pointing to a direction of the airflow.

In some embodiments, as shown in FIG. 13, the fourth deflector structure 60 is disposed in the cavity 11, and the end ③ of the fourth deflector structure 60 is rotatably connected to an inner opening of the inlet 15 on an inner side wall of the top plate 13 by the resilient pivot shaft. In an initial state, the projection ⑤ of the end ④ of the fourth deflector structure 60 along the height direction is located within a projection of the inlet 15 along the height direction; and in a non-initial state, the resilient pivot shaft provides the fourth deflector structure 60 with a restoration force for restoring the fourth deflector structure 60 to the initial state.

In some embodiments, in the initial state, a projection of a free end of the fourth deflector structure that is not connected to the top plate along the height direction is located within the projection of the inlet along the height direction, i.e., the fourth deflector structure is directed to a side wall of the cavity along a direction from a fixed end to the free end. Therefore, when the cooling air enters the inlet, the airflow is directed to the side wall of the cavity by the fourth deflector structure to allow more flow of the cooling air to the bottom of the cavity. At this time, when the fourth deflector structure is blown, the free end of the fourth deflector structure is rotated along a direction away from the side wall of the cavity. After stopping the air intake or reducing the air intake, the force on the resilient pivot shaft decreases and starts to return, and the return force drives the fourth deflector structure to rotate to the initial state (i.e., the free end of the fourth deflector structure is rotated along a direction close to the side wall of the cavity).

In some embodiments of the present disclosure, by providing the fourth deflector structure that is freely rotated at the inlet, the flow direction of a heat dissipation medium can be adaptively adjusted, thereby improving the adaptive capability of a heat dissipation system.

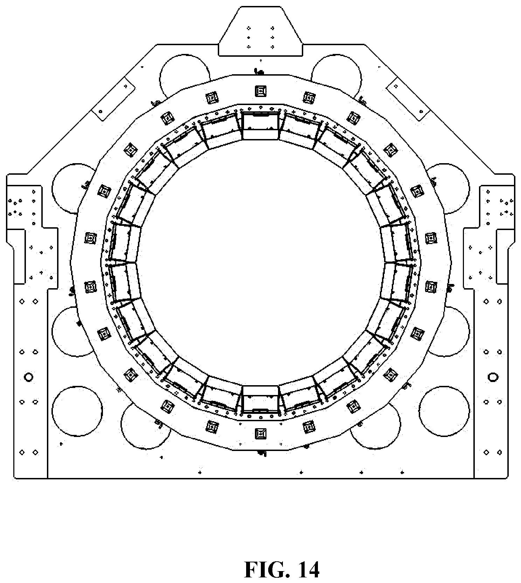

FIG. 14 is a schematic diagram illustrating an exemplary structure of a positron emission tomography (PET) scanning system according to some embodiments of the present disclosure.

In some embodiments, the PET scanning system includes a gantry, a heat dissipation system, and a processor. A plurality of PET detection components are arranged in an array along a circumferential direction on the gantry (as shown in FIG. 14). The heat dissipation system is configured to deliver a heat dissipation medium to the plurality of PET detection components, and the processor is configured to at least control an operation state of the heat dissipation system.

In some embodiments, for any two adjacent PET detection components, side plates of their respective shells are arranged opposite to each other, and orientations of an inlet and an outlet on their respective top plates are both perpendicular to an axial direction of the gantry.

In some embodiments, a long side direction of the PET detection component is parallel to the axial direction of the gantry, and a short side direction of the PET detection component is perpendicular to the axial direction of the gantry.

An axis of the gantry is a central axis that is perpendicular to a circumferential section of the gantry and crosses over the center of the gantry.

According to FIG. 1, FIG. 2, and FIG. 14, among the plurality of PET detection components in an array along the circumferential direction of the gantry, for any two adjacent PET detection components, side plates of their respective shells are arranged opposite to each other. For example, a rear plate of a shell of a PET detection component may be arranged opposite to a front plate of a shell of an adjacent PET detection component.

In some embodiments, for any two adjacent PET detection components, an inlet and an outlet on top plates of their respective shells are oriented perpendicularly to the axial direction of the gantry, or an outlet of one PET detection component is disposed on a top plate of its respective shell, and an outlet of another PET detection component is disposed on a side plate of its respective shell, with inlets of the two adjacent PET detection components oriented perpendicular to the axial direction of the gantry.

The heat dissipation system refers to a system used to deliver a heat dissipation medium to the PET detection component. For example, the heat dissipation system may be a chiller or infusion pump.

In some embodiments, the processor may include one or more sub-processing devices (e.g., a single-core processing device or a multi-core processing device). Merely by way of example, the processor may include a central processing unit (CPU), an application-specific integrated circuit (ASIC), etc., or any combination of the above.

In some embodiments, the processor may be configured to control an operation state of the heat dissipation system. The operation state of the heat dissipation system may include start/stop, output efficiency, operation time, or the like, of the heat dissipation system.

In some embodiments, the heat dissipation system includes an air intake cavity and an air outlet cavity. The air intake cavity and an air outlet cavity are sequentially arranged along the axial direction of the gantry with an axis of the gantry as a central axis. Inlets of heat dissipation devices of the plurality of PET detection components are all in communication with the air intake cavity, and outlets of the heat dissipation devices of the plurality of PET detection components are all in communication with the air outlet cavity. A first air storage cavity is disposed between any of the inlets and the air intake cavity, a first opening of the first air storage cavity is in communication with the air intake cavity, and a second opening of the first air storage cavity is in communication with the inlet. A second air storage cavity is provided between any of the outlets and the air outlet cavity, a third opening of the second air storage cavity is in communication with the air outlet cavity, and a fourth opening of the second air storage cavity is in communication with the outlet.

In some embodiments, both the air intake cavity and the air outlet cavity may be an annular cylindrical cavity structure and are configured for air intake and air outlet, respectively. Both the air intake cavity and the air outlet cavity are arranged along the axial direction of the gantry with the axis of the gantry as the central axis. The air intake cavity and the air outlet cavity are annularly fitted around the plurality of PET detection components in an array along the circumferential direction of the gantry.

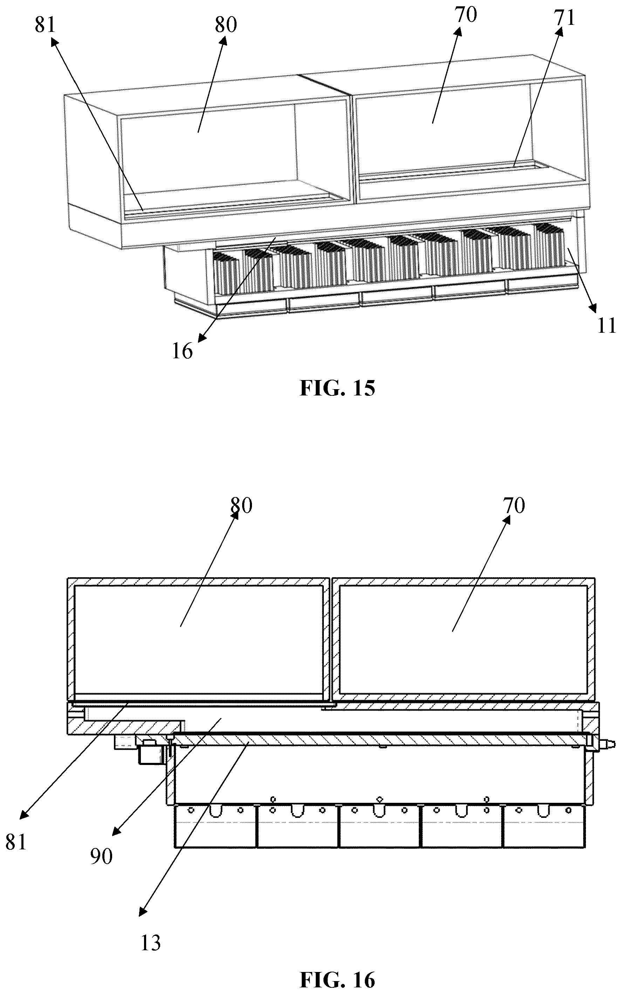

FIG. 15 is a schematic diagram illustrating an exemplary structure of a portion of a heat dissipation system corresponding to one PET detection component according to some embodiments of the present disclosure. FIG. 16 is a schematic diagram illustrating a side-sectional view of a heat dissipation system according to some embodiments of the present disclosure.

In some embodiments, referring to FIG. 15, FIG. 16, and FIG. 2, an air intake cavity 70 is in communication with the inlets 15 of the heat dissipation devices of the plurality of PET detection components 100. A first air storage cavity (not shown in the figures) is disposed between any of the inlets 15 and the air intake cavity 70. The first air storage cavity may be a stepped cavity structure and is formed by the gap between the air intake cavity 70 and the shell 10 of the heat dissipation device. Specifically, the first air storage cavity is a cavity structure with the bottom surface of the air intake cavity 70 serving as its top, the top plate 13 of the shell 10 serving as its bottom, and four side surfaces enclosing the space. The four side surfaces include a left side surface and a right side surface, formed along the width direction of the first air storage cavity (which corresponds to the short side direction of the shell) and along the height direction of the first air storage cavity (which corresponds to the height direction of the shell), respectively, and a front side surface and a rear side surface, formed along the length direction of the first air storage cavity (which corresponds to the long side direction of the shell) and the width direction of the first air storage cavity, respectively.

A first opening 71 is an air inlet of the first air storage cavity. A second opening (not shown in the figures) is an air outlet of the first air storage cavity. In some embodiments, the first opening 71 of the first air storage cavity is located on the bottom surface of the air intake cavity 70 and is in communication with the air intake cavity 70. A cooling medium (such as cold air) inside the air intake cavity 70 enters the first air storage cavity through the first opening 71. The second opening of the first air storage cavity is located on the top plate 13 of the shell 10 and is in communication with the inlet 15 on the top plate 13 of the shell 10 of the heat dissipation device. A cooling medium inside the first air storage cavity enters the cavity 11 of the heat dissipation device through the second opening.

Along the short side direction of the shell, the size of the first air storage cavity is larger than the dimension of the inlet. Along the axial direction of the gantry, the size of the first opening is less than or equal to the size of the air intake cavity, and the size of the second opening is greater than or equal to the size of the inlet.

In some embodiments, referring to FIG. 15, FIG. 16, and FIG. 2, an air outlet cavity 80 is in communication with outlets 16 of the heat dissipation devices of the plurality of PET detection components 100. A second air storage cavity 90 is disposed between any of the outlets 16 and the air outlet cavity 80. The second air storage cavity 90 may be a stepped cavity structure, formed by the gap between the air outlet cavity 80 and the shell 10 of the heat dissipation device. Specifically, the second air storage cavity 90 is a cavity structure with the bottom surface of the air outlet cavity 80 serving as its top, the top plate 13 of the shell 10 serving as its bottom, and four side surfaces enclosing the space. The four side surfaces include a left side surface and a right side surface, formed along the width direction of the second air storage cavity (which corresponds to the short side direction of the shell) and along the height direction of the second air storage cavity (which corresponds to the height direction of the shell), respectively, and a front side surface and a rear side surface, formed along the length direction of the second air storage cavity (which corresponds to the long side direction of the shell) and the width direction of the second air storage cavity, respectively. The width of the second air storage cavity is less than the width of the shell. The first air storage cavity and the second air storage cavity are sequentially arranged along the short side direction of the shell.

A third opening 81 is an air outlet of the second air storage cavity 90. A fourth opening (not shown in the figures) is an air inlet of the second air storage cavity 90. In some embodiments, the third opening 81 of the second air storage cavity 90 is located on the bottom surface of the air outlet cavity 80 and is in communication with the air outlet cavity 80.

In some embodiments, along the short side direction of the shell, the size of the second air storage cavity is larger than the size of the outlet. Along the axial direction of the gantry, the size of the third opening is less than or equal to the size of the air outlet cavity, and the size of the fourth opening is greater than or equal to the size of the outlet.

In some embodiments of the present disclosure, the arrangement of the air storage cavities allows the air intake cavity and the air outlet cavity to cooperate with the inlets and outlets, thereby achieving a short heat dissipation path while enabling the heat dissipation device to have a larger air intake and outlet volume, thereby enhancing the heat dissipation effect.

In some embodiments, a positron emission tomography (PET) detection system, comprises a gantry with a cylindrical structure, a front cover plate, a rear cover plate, a plurality of PET detectors, and a heat dissipation device. The front cover plate is disposed at a front end of the gantry along an axial direction of the cylindrical structure, the front cover plate being provided with an air intake vent; the rear cover plate is disposed at a rear end of the gantry along the axial direction of the cylindrical structure, the rear cover plate being provided with an air outlet vent; the plurality of PET detectors are mounted on the gantry along a circumferential direction of the gantry, each of the plurality of PET detectors extending along a direction from the front cover plate to the rear cover plate; and the heat dissipation device is connected to the plurality of PET detectors. The heat dissipation device includes a shell, the shell forming a cavity, and the cavity being in communication with the air intake vent and the air outlet vent; a cooling component, including a heat-conducting plate and a plurality of cooling members, the heat-conducting plate being thermally coupled with the PET detectors, the plurality of cooling members being arranged at intervals on the heat-conducting plate, and the plurality of cooling members extending along a height direction of the shell; and the cavity forming a flow guide channel for guiding a heat dissipation medium to enter the cavity through the air intake vent to flow along a short side direction of the cavity and flow out of the cavity through the air outlet vent, thereby removing heat from the cooling component. The short side direction refers to a direction of a short side of the shell on a cross-section perpendicular to the height direction.

In some embodiments, with reference to FIG. 14, the front end and the rear end of the gantry refer to a front end and a rear end of the gantry that are perpendicular to an axis of the cylindrical structure. The front end and the rear end are arranged opposite each other along an axial direction of the gantry, and a direction from the front end of the gantry to the rear end of the gantry (i.e., a direction from the front cover plate to the rear cover plate) is parallel to the axial direction of the gantry.

In some embodiments, the front end of the gantry is provided with the front cover plate, and the rear end of the gantry is provided with the rear cover plate. The front cover plate of the gantry may be used to form an air intake cavity, and the rear cover plate may be used to form an air outlet cavity. An air intake vent on the front cover plate may serve as a first opening, and an air outlet vent on the rear cover plate may serve as a third opening, and the air intake vent and the air outlet vent both are in communication with the cavity formed by the shell of the heat dissipation device. A heat dissipation medium (e.g., cold air) flows into the cavity through the air intake vent on the front cover plate and then flows out of the cavity through the air outlet vent on the rear cover plate. For further details regarding the air intake cavity, the air outlet cavity, the first opening, and the third opening, please refer to FIG. 15 and FIG. 16, and the related descriptions thereof.

In some embodiments, the plurality of PET detectors are sequentially mounted on the gantry along a circumferential direction of the gantry, with each of the plurality of PET detectors extending along the direction from the front cover plate to the rear cover plate. The direction from the front cover plate to the rear cover plate is the same as a long side direction of the PET detection component, and also the same as the long side direction of the shell, i.e., a direction parallel to the axial direction of the gantry.

In some embodiments, the heat-conducting plate is thermally coupled with the plurality of PET detectors, and the plurality of cooling members are arranged in an interlaced array and are fixedly connected to the heat-conducting plate. The plurality of cooling members extend along the height direction of the cavity.

A cavity is formed inside the shell and the cavity is in communication with both the air intake vent and the air outlet vent. After the heat dissipation medium enters the cavity through the air intake vent, the flow guide channel formed by the cavity guides the heat dissipation medium to flow along the short side direction of the cavity and flows out of the cavity through the air outlet vent. During this process, the heat dissipation medium comes into full contact with the cooling members to absorb and carry away heat. The short side direction of the cavity is the same as a short side direction of the shell.

In some embodiments, the cavity includes a plurality of sub-cavities, the plurality of sub-cavities are separated from each other, and the plurality of sub-cavities are arranged along the direction from the front cover plate to the rear cover plate. The plurality of sub-cavities may be separated by baffles.

For further details regarding the above content, please refer to FIG. 1 to FIG. 7 and the related descriptions thereof.

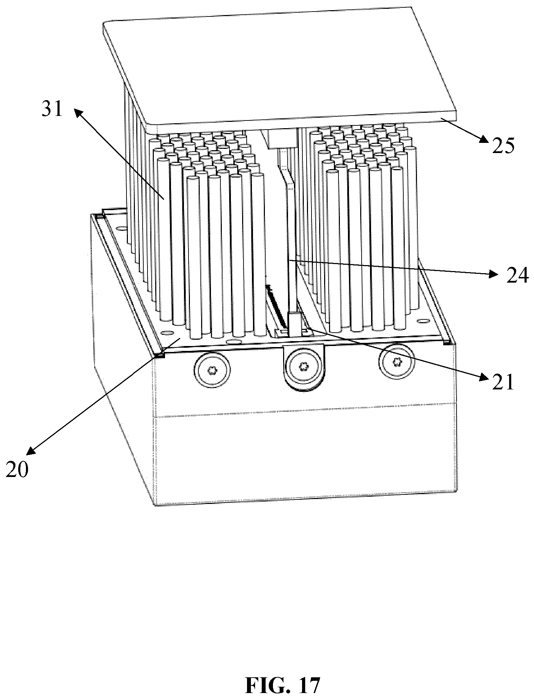

FIG. 17 is a schematic diagram illustrating an exemplary connection structure of a signal transmission board according to some embodiments of the present disclosure. In some embodiments, as shown in FIG. 17, a signal transmission board 24 is disposed inside a cavity, with one end of the signal transmission board 24 connected to the plurality of PET detectors and another end connected to a processor.

A processor board 25 may be a circuit board integrated with a processor and a Field-Programmable Gate Array (FPGA), and the signal transmission board 24 may be a circuit board with signal transmission functionality.

In some embodiments, the signal transmission board 24 passes through the through-slot 21 on the heat-conducting plate 20, with one upward end connected to the processor on the processor board 25 along the height direction of the cavity and a downward end connected to the PET detectors (not shown in the figure) located on a side of the heat-conducting plate 20 back away from the cavity. An upward direction along the height direction of the cavity refers to a pointing direction of the cooling members extending along the height direction of the cavity.

The signal transmission board 24 is the same as a vertical plate and the processor board 25 is the same as a horizontal plate. More descriptions regarding the vertical plate and the horizontal plate can be found in FIGs. 3 and 6, and the related descriptions thereof.

In some embodiments of the present disclosure, the PET detector system can improve the heat transfer efficiency between the heat dissipation medium and the cooling members, effectively shorten the flow path of the heat dissipation medium, reduce a temperature gradient on the circuit board, and enhance the heat dissipation efficiency as well as the operational capability of the PET detectors.

Some features, structures, or characteristics of one or more embodiments of the present disclosure may be suitably combined.

Some embodiments use numbers to describe the number of components, attributes, and it should be understood that such numbers used in the description of the embodiments are modified in some examples by the modifiers "about", "approximately", or "substantially". Unless otherwise noted, the terms "about", "approximately", or "substantially" indicates that a ±20% variation in the stated number is allowed. Correspondingly, in some embodiments, the numerical parameters used in the present disclosure and claims are approximations, which can change depending on the desired characteristics of individual embodiments. In some embodiments, the numerical parameters should take into account the specified number of valid digits and employ general place-keeping. While the numerical domains and parameters used to confirm the breadth of their ranges in some embodiments of the present disclosure are approximations, in specific embodiments, such values are set to be as precise as possible within the range of feasibility.

In the event of any inconsistency or conflict between the descriptions, definitions, and/or the use of terms in the materials cited in the present disclosure and what is stated in the present disclosure, the descriptions, definitions, and/or the use of terms in the present disclosure shall prevail.

## Claims

1. A positron emission tomography (PET) detection component, comprising at least one PET detector and a heat dissipation device,
wherein the PET detector includes an electronic component and crystals that are interconnected; and
the heat dissipation device includes:
a shell (10), a cavity (11) being disposed inside the shell (10), and a first portion of the PET detector being mounted inside the cavity (11); wherein the shell (10) includes a side plate (12), a top plate (13), and a cover shell (14);
a cooling component, including a heat-conducting plate (20) and a plurality of cooling members (31), the heat-conducting plate (20) being located at a bottom of the cavity (11), the plurality of cooling members (31) being arranged at intervals on the heat-conducting plate (20), and the plurality of cooling members (31) extending along a height direction of the shell (10); wherein a top surface of the cavity (11) is the top plate (13), a bottom surface of the cavity (11) is the heat-conducting plate (20), and a side surface of the cavity (11) is the side plate (12); and
an inlet (15) and an outlet (16) disposed on two opposites along a short side direction of the shell (10), respectively, the inlet (15) and the outlet (16) being in communication with the cavity (11), and the short side direction being a direction of a short side of the shell (10) on a cross-section perpendicular to the height direction;
wherein the inlet (15) and the outlet (16) are located on two opposite long sides of the top plate (13), respectively, and the inlet (15) and the outlet (16) are configured as an elongated shape; and
a second portion of the PET detector being connected to a side of the heat-conducting plate (20) back away from the cavity (11), and the crystals being disposed in the second portion of the PET detector.

2. The PET detection component of claim 1, wherein the cavity (11) includes a plurality of sub-cavities, the plurality of sub-cavities are separated from each other, the plurality of sub-cavities are disposed along a long side direction of the shell (10), the heat-conducting plate (20) is equipped with a plurality of groups of heat dissipation units (30), each group of heat dissipation units (30) includes at least one cooling member (31), at least two groups of heat dissipation units (30) are disposed in each sub-cavity, and any one of the plurality of sub-cavities is in communication with the inlet (15) and the outlet (16).

3. The PET detection component of claim 2, wherein each group of heat dissipation units (30) includes a plurality of arrays of cooling members (31) arranged in a staggered manner,
the heat-conducting plate (20) is provided with at least one through-slot (21), the at least one through-slot (21) is arranged along the long side direction of the shell (10), the through-slot (21) extends along the short side direction of the shell (10), two sides of the through-slot (21) are equipped with at least one group of heat dissipation units (30), respectively, and the first portion of the PET detector is mounted in the through-slot (21); and
a plurality of air baffles (22) are disposed inside the cavity (11), and at least one of the plurality of air baffles (22) is disposed between any two adjacent sub-cavities.

4. The PET detection component of claim 1, wherein the shell (10) includes a plurality of side plates (12), the top plate (13) is disposed over a top of the plurality of side plates (12), the heat-conducting plate (20) is connected to a bottom of the plurality of side plates (12), and a gap exists between the top plate (13) and a free end of the cooling member (31) away from the heat-conducting plate (20).

5. The PET detection component of any one of claims 1-4, wherein the cover shell (14) is disposed on the side of the heat-conducting plate (20) back away from the cavity (11), the second portion of the PET detector is mounted inside the cover shell (14), and the second portion is connected to the heat-conducting plate (20).

6. The PET detection component of any one of claims 1-5, wherein an outer opening of the inlet (15) on an outer side wall of the top plate (13) is provided with a trumpet-shaped first deflector structure, and the outer opening of the inlet (15) is located at a small end of the trumpet-shaped first deflector structure, the trumpet-shaped first deflector structure refers to a conical structure that gradually narrows from a large enc to the small end.

7. The PET detection component of claim 6, wherein the first deflector structure includes two deflector plates (40), the two deflector plates (40) are disposed opposite to each other so that the first deflector structure is trumpet-shaped,
each of the deflector plates (40) includes a frame and a plurality of deflector blades arranged in parallel, and the plurality of deflector blades are rotationally connected to the frame; and/or
both of the two deflector plates (40) are rotationally connected to the top plate (13).

8. The PET detection component of any one of claims 1-7, wherein an outer opening of the outlet (16) on an outer side wall of the top plate (13) is provided with a trumpet-shaped second deflector structure, and the outer opening of the outlet (16) is located at a large end of the trumpet-shaped second deflector structure.

9. The PET detection component of any one of claims 6-8, wherein:
a third deflector structure (50) is disposed inside the cavity (11), and a projection of one end of the third deflector structure (50) along the height direction is located within a projection of the inlet (15) along the height direction; or
a fourth deflector structure (60) is disposed inside the cavity (11), one end of the fourth deflector structure (60) is rotatably connected to an inner opening of the inlet (15) on an inner side wall of the top plate (13) by a resilient pivot shaft; in an initial state, a projection of another end of the fourth deflector structure (60) along the height direction is located within a projection of the inlet (15) along the height direction; and in a non-initial state, the resilient pivot shaft provides a restoration force to the fourth deflector structure (60) to return to the initial state.

10. A positron emission tomography (PET) scanning system, comprising a plurality of PET detection components of claim 1, and further comprising:
a gantry, mounted with the plurality of PET detection components in an array along a circumferential direction of the gantry;
a heat dissipation system, configured to deliver a heat dissipation medium to the plurality of PET detection components; and
a processor, configured to at least control an operation state of the heat dissipation system.

11. The PET scanning system of claim 10, wherein for any two adjacent PET detection components, side plates (12) of their respective shells (10) are arranged opposite to each other, and orientations of an inlet (15) and an outlet (16) on their respective top plates (13) are both perpendicular to an axial direction of the gantry.

12. The PET scanning system of claim 11, wherein a long side direction of the PET detection components is parallel to the axial direction of the gantry, and a short side direction of the PET detection components is perpendicular to the axial direction of the gantry.

13. The PET scanning system of claim 10 or 11, wherein the heat dissipation system includes an air intake cavity (70) and an air outlet cavity (80), the air intake cavity (70) and the air outlet cavity (80) are sequentially arranged along an axial direction of the gantry with an axis of the gantry as a central axis, inlets (15) of heat dissipation devices of the plurality of PET detection components are all in communication with the air intake cavity (70), and outlets of the heat dissipation devices of the plurality of PET detection components are all in communication with the air outlet cavity (80),
a first air storage cavity is disposed between any of the inlets (15) and the air intake cavity (70), a first opening (71) of the first air storage cavity is in communication with the air intake cavity (70), and a second opening of the first air storage cavity is in communication with the inlet (15); and
a second air storage cavity is disposed between any of the outlets (16) and the air outlet cavity (80), a third opening (81) of the second air storage cavity is in communication with the air outlet cavity (80), and a fourth opening of the second air storage cavity is in communication with the outlet (16).

## Patentansprüche

1. Positronen-Emissions-Tomographie (PET)-Detektionskomponente, umfassend mindestens einen PET-Detektor und eine Wärmeableitungsvorrichtung,
wobei der PET-Detektor eine elektronische Komponente und miteinander verbundene Kristalle beinhaltet; und
die Wärmeableitungsvorrichtung beinhaltet:
ein Gehäuse (10), einen Hohlraum (11), der in dem Gehäuse (10) angeordnet ist, und einen ersten Abschnitt des PET-Detektors, der in dem Hohlraum (11) montiert ist; wobei das Gehäuse (10) eine Seitenplatte (12), eine Deckplatte (13) und einen Gehäusedeckel (14) beinhaltet;
eine Kühlkomponente, die eine wärmeleitende Platte (20) und mehrere Kühlelemente (31) beinhaltet, wobei sich die wärmeleitende Platte (20) am Boden des Hohlraums (11) befindet, die mehreren Kühlelemente (31) in Abständen auf der wärmeleitenden Platte (20) angeordnet sind und die mehreren Kühlelemente (31) sich entlang einer Höhenrichtung des Gehäuses (10) erstrecken; wobei eine obere Oberfläche des Hohlraums (11) die Deckplatte (13) ist, eine untere Oberfläche des Hohlraums (11) die wärmeleitende Platte (20) ist und eine Seitenfläche des Hohlraums (11) die Seitenplatte (12) ist; und
einen Einlass (15) und einen Auslass (16), die jeweils an zwei gegenüberliegenden Seiten entlang einer kurzen Seitenrichtung des Gehäuses (10) angeordnet sind, wobei der Einlass (15) und der Auslass (16) mit dem Hohlraum (11) in Verbindung stehen und die kurze Seitenrichtung eine Richtung einer kurzen Seite des Gehäuses (10) in einem Querschnitt senkrecht zur Höhenrichtung ist; wobei der Einlass (15) und der Auslass (16) sich jeweils an zwei gegenüberliegenden langen Seiten der Deckplatte (13) befinden und länglich geformt sind; und
wobei ein zweiter Abschnitt des PET-Detektors mit einer Seite der wärmeleitenden Platte (20) verbunden ist, die von dem Hohlraum (11) abgewandt ist, und die Kristalle in diesem zweiten Abschnitt des PET-Detektors angeordnet sind.

2. PET-Detektionskomponente nach Anspruch 1, wobei der Hohlraum (11) mehrere Teilhohlräume aufweist, wobei die mehreren Teilhohlräume voneinander getrennt sind, die mehreren Teilhohlräume entlang einer langen Seitenrichtung des Gehäuses (10) angeordnet sind, die wärmeleitende Platte (20) mit mehreren Gruppen von Wärmeableitungseinheiten (30) ausgestattet ist, wobei jede Gruppe von Wärmeableitungseinheiten (30) mindestens ein Kühlelement (31) beinhaltet, mindestens zwei Gruppen von Wärmeableitungseinheiten (30) in jedem Teilhohlraum angeordnet sind und jeder der mehreren Teilhohlräume mit dem Einlass (15) und dem Auslass (16) in Verbindung steht.

3. PET-Detektionskomponente nach Anspruch 2, wobei jede Gruppe von Wärmeableitungseinheiten (30) mehrere Anordnungen von Kühlelementen (31) beinhaltet, die versetzt angeordnet sind;
die wärmeleitende Platte (20) mit mindestens einem Durchgangsschlitz (21) versehen ist, der mindestens eine Durchgangsschlitz (21) entlang der langen Seitenrichtung des Gehäuses (10) angeordnet ist und sich entlang der kurzen Seitenrichtung des Gehäuses (10) erstreckt, sich an zwei Seiten des Durchgangsschlitzes (21) jeweils mindestens eine Gruppe von Wärmeableitungseinheiten (30) befindet und der erste Abschnitt des PET-Detektors im Durchgangsschlitz (21) montiert ist; und
mehrere Luftleitbleche (22) im Inneren des Hohlraums (11) angeordnet sind und mindestens eines der mehreren Luftleitbleche (22) sich zwischen je zwei benachbarten Teilhohlräumen befindet.

4. PET-Detektionskomponente nach Anspruch 1, wobei das Gehäuse (10) mehrere Seitenplatten (12) beinhaltet, die Deckplatte (13) über einer Oberseite der mehreren Seitenplatten (12) angeordnet ist, die wärmeleitende Platte (20) mit einem Boden der mehreren Seitenplatten (12) verbunden ist und zwischen der Deckplatte (13) und einem freien Ende des Kühlelements (31), das von der wärmeleitenden Platte (20) abgewandt ist, ein Spalt vorhanden ist.

5. PET-Detektionskomponente nach einem der Ansprüche 1-4, wobei der Gehäusedeckel (14) auf der von dem Hohlraum (11) abgewandten Seite der wärmeleitenden Platte (20) angeordnet ist, der zweite Abschnitt des PET-Detektors innerhalb des Gehäusedeckels (14) montiert ist und der zweite Abschnitt mit der wärmeleitenden Platte (20) verbunden ist.

6. PET-Detektionskomponente nach einem der Ansprüche 1-5, wobei eine äußere Öffnung des Einlasses (15) an einer äußeren Seitenwand der Deckplatte (13) mit einer trompetenförmigen ersten Ablenkstruktur versehen ist und sich die äußere Öffnung des Einlasses (15) an einem schmalen Ende der trompetenförmigen ersten Ablenkstruktur befindet, wobei sich die trompetenförmige erste Ablenkstruktur auf eine konische Struktur bezieht, die sich von einem breiten Ende zum schmalen Ende hin allmählich verjüngt.

7. PET-Detektionskomponente nach Anspruch 6, wobei die erste Ablenkstruktur zwei Ablenkplatten (40) beinhaltet, die zwei Ablenkplatten (40) einander gegenüberliegend angeordnet sind, sodass die erste Ablenkstruktur trompetenförmig ist,
jede der Ablenkplatten (40) einen Rahmen und mehrere parallel angeordnete Ablenkblätter beinhaltet und die mehreren Ablenkblätter drehbar mit dem Rahmen verbunden sind; und/oder
die beiden Ablenkplatten (40) drehbar mit der Deckplatte (13) verbunden sind.

8. PET-Detektionskomponente nach einem der Ansprüche 1-7, wobei eine äußere Öffnung des Auslasses (16) an einer äußeren Seitenwand der Deckplatte (13) mit einer trompetenförmigen zweiten Ablenkstruktur versehen ist und sich die äußere Öffnung des Auslasses (16) an einem breiten Ende der trompetenförmigen zweiten Ablenkstruktur befindet.

9. PET-Detektionskomponente nach einem der Ansprüche 6-8, wobei:
eine dritte Ablenkstruktur (50) innerhalb des Hohlraums (11) angeordnet ist und eine Projektion eines Endes der dritten Ablenkstruktur (50) in Höhenrichtung sich innerhalb einer Projektion des Einlasses (15) in Höhenrichtung befindet; oder
eine vierte Ablenkstruktur (60) im Inneren des Hohlraums (11) angeordnet ist, ein Ende der vierten Ablenkstruktur (60) über eine elastische Schwenkachse drehbar mit einer inneren Öffnung des Einlasses (15) an einer inneren Seitenwand der Deckplatte (13) verbunden ist, in einem Ausgangszustand sich eine Projektion eines anderen Endes der vierten Ablenkstruktur (60) in Höhenrichtung innerhalb einer Projektion des Einlasses (15) in Höhenrichtung befindet und in einem Nicht-Ausgangszustand die elastische Schwenkachse eine Rückstellkraft auf die vierte Ablenkstruktur (60) ausübt, um sie in den Ausgangszustand zurückzuführen.

10. Positronen-Emissions-Tomographie (PET)-Scansystem, umfassend mehrere PET-Detektionskomponenten nach Anspruch 1, und ferner umfassend:
eine Gantry, an der die mehreren PET-Detektionskomponenten in einer Anordnung entlang einer Umfangsrichtung der Gantry montiert sind;
ein Wärmeableitungssystem, das so konfiguriert ist, dass es ein Wärmeableitungsmedium an die mehreren PET-Detektionskomponenten liefert; und
einen Prozessor, der so konfiguriert ist, dass er mindestens einen Betriebszustand des Wärmeableitungssystems steuert.

11. PET-Scansystem nach Anspruch 10, wobei für je zwei benachbarte PET-Detektionskomponenten Seitenplatten (12) ihrer jeweiligen Gehäuse (10) einander gegenüberliegend angeordnet sind und Ausrichtungen eines Einlasses (15) und eines Auslasses (16) auf ihren jeweiligen Deckplatten (13) beide senkrecht zu einer Axialrichtung der Gantry verlaufen.

12. PET-Scansystem nach Anspruch 11, wobei eine lange Seitenrichtung der PET-Detektionskomponenten parallel zur Axialrichtung der Gantry verläuft und eine kurze Seitenrichtung der PET-Detektionskomponenten senkrecht zur axialen Richtung der Gantry verläuft.

13. PET-Scansystem nach Anspruch 10 oder 11, wobei das Wärmeableitungssystem einen Lufteinlasshohlaum (70) und einen Luftauslasshohlaum (80) beinhaltet, der Lufteinlasshohlaum (70) und der Luftauslasshohlaum (80) nacheinander entlang einer Axialrichtung der Gantry mit einer Achse der Gantry als Mittelachse angeordnet sind, Einlässe (15) von Wärmeableitungsvorrichtungen der mehreren PET-Detektionskomponenten alle mit dem Lufteinlasshohlaum (70) in Verbindung stehen und Auslässe der Wärmeableitungsvorrichtungen der mehreren PET-Detektionskomponenten alle mit dem Luftauslasshohlaum (80) in Verbindung stehen,
zwischen einem der Einlässe (15) und dem Lufteinlasshohlaum (70) ein erster Luftspeicherhohlraum angeordnet ist, wobei eine erste Öffnung (71) des ersten Luftspeicherhohlraums mit dem Lufteinlasshohlaum (70) in Verbindung steht und eine zweite Öffnung des ersten Luftspeicherhohlraums mit dem Einlass (15) in Verbindung steht; und
zwischen einem der Auslässe (16) und dem Luftauslasshohlaum (80) ein zweiter Luftspeicherhohlraum angeordnet ist, eine dritte Öffnung (81) des zweiten Luftspeicherhohlraums mit dem Luftauslasshohlaum (80) in Verbindung steht und eine vierte Öffnung des zweiten Luftspeicherhohlraums mit dem Auslass (16) in Verbindung steht.

## Revendications

1. Composant de détection par tomographie par émission de positons (PET), comprenant au moins un détecteur PET et un dispositif de dissipation de chaleur,
dans lequel le détecteur PET inclut un composant électronique et des cristaux interconnectés ; et
le dispositif de dissipation de chaleur inclut :
une coque (10), une cavité (11) disposée à l'intérieur de la coque (10) et une première partie du détecteur PET montée à l'intérieur de la cavité (11) ; dans lequel la coque (10) inclut une plaque latérale (12), une plaque supérieure (13) et une coque de recouvrement (14) ;
un composant de refroidissement incluant une plaque conductrice de chaleur (20) et une pluralité d'organes de refroidissement (31), la plaque conductrice de chaleur (20) étant située à un fond de la cavité (11), la pluralité d'organes de refroidissement (31) étant agencés à des intervalles sur la plaque conductrice de chaleur (20) et la pluralité d'organes de refroidissement (31) s'étendant le long d'une direction de hauteur sur la coque (10) ; dans lequel une surface supérieure de la cavité (11) est la plaque supérieure (13), une surface inférieure de la cavité (11) est la plaque conductrice de chaleur (20) et une surface latérale de la cavité (11) est la plaque latérale (12) ; et
une entrée (15) et une sortie (16) disposées respectivement sur deux côtés opposés dans une direction de côté court de la coque (10), l'entrée (15) et la sortie (16) étant en communication avec la cavité (11), et la direction de côté court étant une direction d'un côté court de la coque (10) sur une section transversale perpendiculaire à la direction de hauteur ; dans lequel l'entrée (15) et la sortie (16) sont situées respectivement sur deux côtés longs opposés de la plaque supérieure (13), et l'entrée (15) et la sortie (16) sont configurées en forme allongée ; et
une seconde partie du détecteur PET étant reliée à un côté de la plaque conductrice de chaleur (20) à l'opposé de la cavité (11), et les cristaux étant disposés dans la seconde partie du détecteur PET.

2. Composant de détection PET selon la revendication 1, dans lequel la cavité (11) inclut une pluralité de sous-cavités, la pluralité de sous-cavités sont séparées les unes des autres, la pluralité de sous-cavités sont disposées le long d'une direction de côté long de la coque (10), la plaque conductrice de chaleur (20) est équipée d'une pluralité de groupes d'unités de dissipation de chaleur (30), chaque groupe d'unités de dissipation de chaleur (30) inclut au moins un organe de refroidissement (31), au moins deux groupes d'unités de dissipation de chaleur (30) sont disposés dans chaque sous-cavité, et l'une quelconque de la pluralité de sous-cavités est en communication avec l'entrée (15) et la sortie (16).

3. Composant de détection PET selon la revendication 2, dans lequel chaque groupe d'unités de dissipation de chaleur (30) inclut une pluralité de réseaux d'organes de refroidissement (31) agencés en quinconce ;
la plaque conductrice de chaleur (20) est munie d'au moins une fente traversante (21), la au moins une fente traversante (21) est agencée le long d'une direction de côté long de la coque (10), la fente traversante (21) s'étend le long de la direction de côté court de la coque (10), deux côtés de la fente traversante (21) sont équipés respectivement d'au moins un groupe d'unités de dissipation de chaleur (30), et la première partie du détecteur PET est montée dans la fente traversante (21) ;
une pluralité de déflecteurs d'air (22) sont disposés à l'intérieur de la cavité (11), et au moins un de la pluralité de déflecteurs d'air (22) est disposé entre deux sous-cavités adjacentes.

4. Composant de détection PET selon la revendication 1, dans lequel la coque (10) inclut une pluralité de plaques latérales (12), la plaque supérieure (13) est disposée au-dessus de la pluralité de plaques latérales (12), la plaque conductrice de chaleur (20) est reliée à un fond de la pluralité de plaques latérales (12), et un espace existe entre la plaque supérieure (13) et une extrémité libre de l'organe de refroidissement (31) éloignée de la plaque conductrice de chaleur (20).

5. Composant de détection PET selon l'une quelconque des revendications 1-4, dans lequel la coque de recouvrement (14) est disposée du côté de la plaque conductrice de chaleur (20) à l'opposé de la cavité (11), la seconde partie du détecteur PET est montée à l'intérieur de la coque de recouvrement (14), et la seconde partie est reliée à la plaque conductrice de chaleur (20).

6. Composant de détection PET selon l'une quelconque des revendications 1-5, dans lequel une ouverture extérieure de l'entrée (15) sur une paroi latérale extérieure de la plaque supérieure (13) est munie d'une première structure de déflecteur en forme de trompette, et l'ouverture extérieure de l'entrée (15) est située à une petite extrémité de la première structure de déflecteur en forme de trompette, la première structure de déflecteur en forme de trompette se rapporte à une structure conique qui se rétrécit progressivement d'une grande extrémité à la petite extrémité.

7. Composant de détection PET selon la revendication 6, dans lequel la première structure de déflecteur inclut deux plaques de déflecteur (40), les deux plaques de déflecteur (40) étant disposées face à face de sorte que la première structure de déflecteur soit en forme de trompette,
chacune des plaques de déflecteur (40) inclut un cadre et une pluralité de lames de déflecteur agencées en parallèle, et la pluralité de lames de déflecteur sont reliées de manière rotative au cadre ; et/ou
les deux plaques de déflecteur (40) sont reliées en rotation à la plaque supérieure (13).

8. Composant de détection PET selon l'une quelconque des revendications 1-7, dans lequel une ouverture extérieure de la sortie (16) sur une paroi latérale extérieure de la plaque supérieure (13) est munie d'une deuxième structure de déflecteur en forme de trompette, et l'ouverture extérieure de la sortie (16) est située à une grande extrémité de la deuxième structure de déflecteur en forme de trompette.

9. Composant de détection PET selon l'une quelconque des revendications 6-8, dans lequel :
une troisième structure de déflecteur (50) est disposée à l'intérieur de la cavité (11), et une projection d'une extrémité de la troisième structure de déflecteur (50) le long de la direction de hauteur est située à l'intérieur d'une projection de l'entrée (15) le long de la direction de hauteur ; ou
une quatrième structure de déflecteur (60) est disposée à l'intérieur de la cavité (11), une extrémité de la quatrième structure de déflecteur (60) est reliée de manière rotative à une ouverture intérieure de l'entrée (15) sur une paroi latérale intérieure de la plaque supérieure (13) par un arbre de pivot élastique ; dans un état initial, une projection de l'autre extrémité de la quatrième structure de déflecteur (60) le long de la direction de hauteur est située à l'intérieur d'une projection de l'entrée (15) le long de la direction de hauteur ; et dans un état non initial, l'arbre de pivot élastique fournit une force de rappel à la quatrième structure de déflecteur (60) pour revenir à l'état initial.

10. Système de balayage de tomographie par émission de positons (PET), comprenant une pluralité de composants de détection PET selon la revendication 1, et comprenant en outre :
un portique, équipé de la pluralité de composants de détection PET disposés en réseau le long d'une direction circonférentielle du portique ;
un système de dissipation thermique, configuré pour acheminer un fluide caloporteur vers la pluralité de composants de détection PET ; et
un processeur, configuré pour commander au moins un état de fonctionnement du système de dissipation de chaleur.

11. Système de balayage PET selon la revendication 10, dans lequel, pour deux composants de détection PET adjacents quelconques, des plaques latérales (12) de leurs coques (10) respectives sont agencées face à face, et des orientations d'une entrée (15) et d'une sortie (16) sur leurs plaques supérieures (13) respectives sont toutes deux perpendiculaires à une direction axiale du portique.

12. Système de balayage PET selon la revendication 11, dans lequel une direction de côté long des composants de détection PET est parallèle à la direction axiale du portique, et une direction de côté court des composants de détection PET est perpendiculaire à la direction axiale du portique.

13. Système de balayage PET selon la revendication 10 ou 11, dans lequel le système de dissipation de chaleur inclut une cavité d'entrée d'air (70) et une cavité de sortie d'air (80), la cavité d'entrée d'air (70) et la cavité de sortie d'air (80) sont agencées séquentiellement le long d'une direction axiale du portique avec un axe du portique comme axe central, des entrées (15) de dispositifs de dissipation de chaleur de la pluralité de composants de détection PET sont toutes en communication avec la cavité d'entrée d'air (70), et des sorties des dispositifs de dissipation de chaleur de la pluralité de composants de détection PET sont toutes en communication avec la cavité de sortie d'air (80),
une première cavité de stockage d'air est disposée entre l'une quelconque des entrées (15) et la cavité d'entrée d'air (70), une première ouverture (71) de la première cavité de stockage d'air est en communication avec la cavité d'entrée d'air (70), et une seconde ouverture de la première cavité de stockage d'air est en communication avec l'entrée (15) ; et
une seconde cavité de stockage d'air est disposée entre l'une quelconque des sorties (16) et la cavité de sortie d'air (80), une troisième ouverture (81) de la seconde cavité de stockage d'air est en communication avec la cavité de sortie d'air (80), et une quatrième ouverture de la seconde cavité de stockage d'air est en communication avec la sortie (16).
